# EUROPEAN PATENT SPECIFICATION

(11) **EP 2 760 487 B1**
(45) Date of publication and mention of the grant of the patent: **21.08.2019**
(21) Application number: 12836554.1
(22) Date of filing: 05.09.2012
(51) Int. Cl.: A61L 15/22, A61F 13/15, A61L 15/28, A61F 13/00

(54) **WOUND DRESSINGS AND RELATED METHODS THEREFOR**
WUNDVERBÄNDE UND ZUGEHÖRIGE VERFAHREN
PANSEMENTS ET PROCÉDÉS ASSOCIÉS

(30) Priority: 30.09.2011 US 201113250210
(43) Date of publication of application: 06.08.2014
(73) Proprietor: KPR U.S., LLC, Mansfield, MA 02048 (US)
(72) Inventor: PATEL, Harish, A., Norfolk, MA 02056 (US); MOGHE, Ajit, K., Bellingham, MA 02019 (US)
(74) Representative: Prock, Thomas
(86) International application number: PCT/US2012/053741
(87) International publication number: WO 2013/048681

(56) References cited:
- DE-U1- 7 835 711
- US-A- 4 236 550
- US-A- 6 140 257
- US-A1- 2003 120 193
- US-A1- 2003 190 853
- US-A1- 2003 190 853
- US-A1- 2003 191 423
- US-A1- 2009 308 404
- US-A1- 2010 042 034

## Description

### FIELD

The present disclosure relates to woven fabrics, and more particularly, to wound dressings with bast fibers, cellulosic fibers, and alternative polymeric fibers having preferred or customized properties.

### BACKGROUND

Wound dressings have been used in the medical industry to protect and/or facilitate healing of open wounds. Wound dressings may include an absorbent pad to provide both cushioning to a wound site and assistance in the collection of exudates from the wound.

Woven gauze fabric has been used as a wound dressing to absorb wound exudates and to protect the wound from unwanted environmental factors. Such fabric is loosely woven and includes yarns made of cellulosic fibers, such as cotton and viscose rayon. The absorbency of the gauze fabric is a function of absorbent capacity of the individual fibers in the yarn and the absorption capacity of the interstices within the yarn and between the successive yarns. Therefore, the gauze fabric structure, as a whole, provides an optimal substrate to absorb wound exudates.

Wound dressings are generally placed over a wound to protect and promote healing of the wound. In the case of exuding wounds, such as pressure sores, ulcers, and burns, it is customary to provide a dressing having an absorbent material for absorbing at least a portion of the wound exudates as it is produced. Absorbing exudates promotes healing by removing potentially harmful bacteria from the wound bed, and also prevents damage to the surrounding skin that can be caused by an excessively moist environment.

The absorbent material temporarily stores the excess exudates until such time as they may be removed, usually as the dressing is periodically replaced with a new dressing. Some absorbent materials such as cotton tend to become attached to a healing wound bed and may shed small fibers into the wound that may remain in the wound when the dressing is changed. Removing the dressing and/or stray fibers can be a labor intensive procedure that may further damage the wound, and neglecting to remove stray fibers may cause irritation or form granuloma and otherwise inhibit natural healing of the wound. Examples of wound dressings are found in DE 78 35 711 U1, US 6 140 257 and US 2010/042034.

It would be advantageous to provide a wound dressing with customized properties, such as, for example, increased strength, lower surface lint, lower adhesion to a wound, and/or increased absorbency, to improve wound healing and the cost effectiveness of the wound dressing.

### SUMMARY

In accordance with claim 1 of the present disclosure, a wound dressing comprising a substrate includes first yarns and second yarns, the first yarns including at least one fiber comprising at least one of a cellulosic material or a bast fiber, and the second yarns including at least one fiber comprising an alternative polymeric material, wherein at least one of the first and second yarns includes at least one monofilament bi-component fiber fabricated by co-extruding two distinct polymers.

A wound dressing of the present disclosure may include a substrate including a number of yarns in a warp direction defining a length of the substrate and a number of yarns in a weft direction defining a width of the substrate. At least one yarn in the warp direction is intermingled with at least one yarn in the weft direction. The substrate has at least one yarn including cellulosic fibers and at least one yarn including alternative polymeric fibers.

One aspect of the invention is directed to method of providing a wound dressing comprising providing a wound dressing including first yarns and second yarns, the first yarns including at least one fiber comprising one or more of a material selected from the group consisting of cotton, rayon, flax, lyocell, and combinations thereof, and the second yarns including at least one fiber comprising an alternative polymeric material selected from the group consisting of polyolefins, polyamides, polyesters, and combinations thereof, wherein at least one of the first and second yarns includes at least one monofilament bi-component fiber fabricated by co-extruding two distinct polymers; and sterilizing the wound dressing.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiments given below, serve to explain the principles of the disclosure:
FIGS. 1-5 are schematic illustrations showing various cross-sectional views of one or more embodiments of fibers that can be utilized in one or more wound dressings in accordance with the invention;
FIG. 6 is a schematic illustration showing a perspective view of a partial cross-section of a yarn that can be utilized in accordance with one or more embodiments of the invention;
FIG. 7 is a schematic illustration showing a perspective view of an embodiment of a yarn of a wound dressing in accordance with one or more embodiments of the invention; and
FIGS. 8-16 are schematic illustrations showing various embodiments of wound dressings in accordance with one or more aspects of the invention.

### DETAILED DESCRIPTION

Woven fabrics such as gauze in accordance with the present disclosure are fabricated from a textile including at least two different polymeric fibers. While the substrates, such as fabrics, are discussed herein in terms of wound dressings, it is envisioned that the substrates may be used in connection with a variety of medical and surgical procedures as bandages and surgical sponges, for example. The substrates may be in the form of sheets and patches, as well composite materials such as pledgets, buttresses, and drug delivery devices.

A wound dressing of the present disclosure can be formed from monofilament or multifilament yarns made from at least two different biocompatible, polymeric materials. Suitable materials from which the wound dressing can be made should have the following characteristics: sufficiently strong to avoid tearing of portions thereof; sufficiently inert to avoid foreign body reactions when retained on or in the body for long periods of time; easily sterilized to prevent the introduction of infection when the dressing is placed upon or in the body; and suitably easy handling characteristics for placement in the desired location on the body. The wound dressing should also be sufficiently pliable to conform to a tissue surface, such as a wound, and flex with movement of the tissue.

One of more aspects of the invention can pertain to a wound dressing comprising a substrate comprising first yarns and second yarns. In further aspects of the invention, the wound dressing can consist essentially of first yarns and second yarns. In still other aspects of the invention, the wound dressing can consist of first yarns and second yarns. The first yarns can comprise at least one fiber comprising a cellulosic material or one or more types of cellulosic materials. In other embodiments of the invention, the first yarns can comprise at least one fiber consisting essentially of a cellulosic material or one or more types of cellulosic materials. In further embodiments of the invention, the first yarns can comprise at least one fiber consisting of a cellulosic material or one or more types of cellulosic materials. In some embodiments of the invention, the first yarns can comprise at least one bast fiber or one or more types of bast fibers. In other embodiments of the invention, the first yarn can consist essentially of a bast fiber, or one or more types of bast fibers. In further embodiments of the invention, the first yarn can consist of a bast fiber, or one or more types of bast fibers. In some cases, the first yarns can consist essentially of bast fibers or cellulosic fibers. In some cases, the first yarns can consist of bast fibers or cellulosic fibers. In some cases, the first yarns can consist essentially of bast fibers and cellulosic fibers. In some embodiments of the invention, the second yarns can comprise at least one fiber comprising an alternative polymeric material. In other embodiments of the invention, the second yarn can comprise at least one fiber consisting essentially of an alternative polymeric material or one or more types of alternative polymeric materials. In further embodiments of the invention, the second yarn can comprise at least one fiber consisting of an alternative polymeric material or one or more types of alternative polymeric materials. In some cases, the first yarns can consist essentially of bast fibers and cellulosic fibers. In some cases, the first yarns can consist of bast fibers and cellulosic fibers. In some cases, the second yarns can consist essentially of bast fibers and alternative polymeric materials. In some cases, the second yarns can consist of bast fibers and alternative polymeric materials. The alternative polymeric material is typically in a range of from about 5% to about 65% by weight of the substrate or the wound dressing.

At least one of the first and second yarns comprise at least one bi-component fiber; in other cases, at least one of the first and second yarns can consist essentially of at least one bi-component fiber; and in still other cases, only the second yarns can consist of at least one bi-component fiber. At least a portion of the first yarns can comprise heterogeneous yarns including at least one fiber of a first cellulosic material and at least one fiber of a second cellulosic material. In other cases, the first yarn can consist essentially of heterogeneous yarns with a first cellulosic material and a second cellulosic material. In still other cases, the first yarn can consist of heterogeneous yarns with a first cellulosic material and a second cellulosic material. At least a portion of the second yarns can comprise heterogeneous yarns comprising at least one fiber of a first alternative polymeric material and at least one fiber of a second alternative polymeric material; in some cases, the heterogeneous yarns can consist essentially of at least one fiber of a first alternative polymeric material and at least one fiber of a second alternative polymeric material; and in further cases, the heterogeneous yarns can consist of at least one fiber of a first alternative polymeric material and at least one fiber of a second alternative polymeric material.

The second yarn can comprise at least one blended fiber with a cellulosic material and an alternative polymeric material. In some cases, the second yarn can consist essentially of at least one blended fiber with a cellulosic material and an alternative polymeric material. In still other cases, the second yarn can consist of at least one blended fiber with a cellulosic material and an alternative polymeric material. In some further cases, the second yarn can consist essentially of at least one blended fiber with a bast material and an alternative polymeric material. In still other cases, the second yarn can consist of at least one blended fiber with a bast material and an alternative polymeric material.

At least a portion of the first yarns can be comprised of a cellulosic material selected from the group consisting of cotton, rayon, and combinations thereof. At least a portion of the second yarns can be comprised of an alternative polymeric material is selected from the group consisting of polyolefins, polyamides, polyesters, and combinations thereof. At least a portion of the first yarns can be comprised of bast fibers.

The wound dressing, including for example substrates thereof, can comprise first yarns woven in the warp direction and second yarns woven the weft direction. In other cases, the wound dressing, such as substrates thereof, can consist essentially of first yarns woven in the warp direction and second yarns woven the weft direction. In further cases, the wound dressing, such as substrates thereof, can consist of first yarns woven in the warp direction and second yarns woven the weft direction. In variants of some cases, the wound dressing, such as substrates thereof, can comprise first yarns woven in the weft direction and second yarns woven the warp direction. In other variants, the wound dressing, such as substrates thereof, can consist essentially of first yarns woven in the weft direction and second yarns woven the warp direction. In further variants, the wound dressing, such as substrates thereof, can consist of first yarns woven in the weft direction and second yarns woven the warp direction. The first yarns can comprise cellulosic fibers. In some cases, the first yarns can consist essentially of cellulosic fibers. In other cases, the first yarns can consist of cellulosic fibers. In some still other cases, the first yarns can consist essentially of bast fibers, such as one or more types of bast fibers. In other cases, the first yarns can consist of bast fibers, such as one or more types of bast fibers.

One of more aspects of the invention can pertain to a method of facilitating treating a wound or to a method of providing a wound dressing, comprising providing a substrate for a wound dressing including first yarns and second yarns, the first yarns including at least one fiber comprising a cellulosic material selected from the group consisting of cotton, rayon, and combinations thereof, and the second yarns including at least one fiber comprising an alternative polymeric material selected from the group consisting of polyolefins, polyamides, polyesters, and combinations thereof, and sterilizing the wound dressing. In some embodiments of the invention, the method can comprise weaving first yarns in a warp direction of a substrate; in other embodiments of the invention, the method can comprise weaving first yarns in only a warp direction of the substrate, and in further embodiments of the invention, the method can comprise weaving first yarns in only a weft direction of the substrate. One of more aspects of the invention can pertain to a method of providing a wound dressing or to a method of facilitating treating a wound comprising providing a substrate or a wound dressing including first yarns and second yarns, the first yarns including at least one bast fiber and a cellulosic material selected from the group consisting of cotton, rayon, and combinations thereof, and the second yarns including at least one fiber comprising an alternative polymeric material selected from the group consisting of polyolefins, polyamides, polyesters, and combinations thereof, and sterilizing the wound dressing. In some embodiments of the invention, the method can comprise weaving first yarns in a warp direction of a substrate; in other embodiments of the invention, the method can comprise weaving first yarns in only a warp direction of the substrate, and in further embodiments of the invention, the method can comprise weaving first yarns in only a weft direction of the substrate. The substrate can thus be a woven fabric.

Providing the wound dressing, in accordance with some embodiments of the invention, can comprise weaving at least a portion of the first yarns in a weft direction and at least a portion of the second yarns in the warp direction to produce the substrate, such as a woven fabric, with an amount of the alternative polymeric material in a range of from about 5% to about 65% by weight thereof. In other embodiments of the invention, providing the wound dressing can comprise weaving at least a portion of the first yarns in a warp direction and at least a portion of the second yarns in the weft direction to produce a fabric with an amount of the alternative polymeric material in a range of from about 5% to about 65% by weight thereof.. In further embodiments of the invention, providing the wound dressing can comprise weaving at least a portion of the first yarns consisting essentially of heterogeneous yarns with at least one fiber of a first cellulosic material and at least one fiber of a second cellulosic material with at least a portion of the second yarns consisting essentially of heterogeneous yarns with at least one fiber of a first alternative polymeric material and at least one fiber of a second alternative polymeric material to produce a fabric having an amount of the alternative polymeric material in a range of from about 5% to about 65% by weight thereof. In further embodiments of the invention, providing the wound dressing can comprise weaving at least a portion of the first yarns consisting essentially of heterogeneous yarns with at least one bast fiber and at least one fiber of a cellulosic material with at least a portion of the second yarns consisting essentially of heterogeneous yarns with at least one fiber of a first alternative polymeric material and at least one fiber of a second alternative polymeric material to produce a fabric having an amount of the alternative polymeric material in a range of from about 5% to about 65% by weight thereof. In still further embodiments of the invention, providing the wound dressing can comprise weaving first yarns with the second yarns to produce a woven article, bleaching the woven article, tentering the bleached, woven article, cutting the bleached, woven article to produce a woven fabric, and folding the woven fabric to produce a gauze or dressing. Folding the woven fabric can comprise folding the woven fabric to produce a gauze having any of three plies, four plies, five plies, six plies, eight plies, ten plies, twelve plies, sixteen plies, 24 plies, 32 plies, 48 plies, 50 plies, 144 plies, and 216 plies.

In some embodiments of the invention, the method can comprise weaving first yarns consisting essentially of cotton with second yarns consisting essentially of polyester to produce a fabric with an amount of the polyester in a range of from about 5% to about 65% by weight of the fabric. In some embodiments of the invention, the method can comprise weaving first yarns consisting essentially of bast fibers with second yarns consisting essentially of polyester to produce a fabric with an amount of the polyester in a range of from about 5% to about 65% by weight of the fabric.

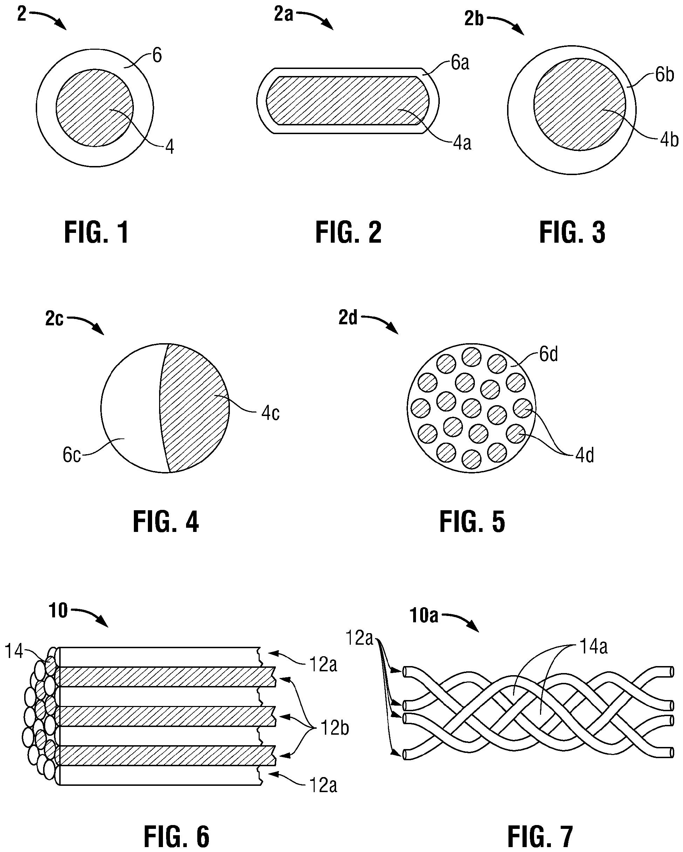

Any of the yarns in any of the various embodiments of the invention may include one or more bi-component fibers. As used herein, a bi-component fiber is a fiber of two polymers which may have different chemical and/or physical properties. As exemplarily illustrated in FIG. 1, a bi-component fiber 2 can include a core 4 comprising, consisting essentially of, or consisting of a first polymer, and a sheath 6 comprising, consisting essentially of, or consisting of a second polymer. Core polymer 4 may be fabricated from a first polymeric material and sheath polymer 6 may be fabricated from a second polymeric material having different characteristics than the first polymeric material.

Bi-component fiber 2 may be a monofilament fiber which is can be fabricated by co-extruding two distinct polymers into a fiber with a concentric sheath-core arrangement as exemplarily illustrated. Fiber 2 may have a round cross-sectional shape including core polymer 4 surrounded by a sheath polymer 6. Other cross-sectional shapes are envisioned, such as a flat cross-sectional shape as illustrated by fiber 2a in FIG. 2, as well as other modified cross-sections geometries. In any of such configurations, the fibers may be coextruded with any desired cross-sectional geometry.

Core polymer 4 and sheath polymer 6 may be concentrically arranged as illustrated in FIG. 1. In other embodiments of the invention, any of the fibers in the various embodiments of the invention can be a bi-component fiber 2b with an eccentric sheath-core arrangement, as illustrated in FIG. 3. Fiber 2b can include an off-center core polymer 4b surrounded by sheath polymer 6b. Further variants can involve bi-component fibers 2c with core polymer 4c and sheath polymer 6c each occupying portions of the outer surface of the fiber 2c, as illustrated in FIG. 4.

Any of the bi-component fiber 2d may also be multifilament fibers spun and processed from microdenier filaments of core polymer 4d, as illustrated in the cross-sectional view of FIG. 5. Further, fiber 2d can be configured in an islands-in-the-sea arrangement wherein two or more discontinuous or non-contiguous islands of core polymer filaments 4d, are surrounded by a continuous or contiguous sea or sheath of polymer 6d. This arrangement may provide for strands of island polymer filaments 4d to be effectively handled by manufacturing equipment to spin and form fiber 2d. Core polymer filaments 4d may be arranged so as to be generally non-intersecting along their length. Although not necessarily parallel, core polymer filaments 4d may be generally free from entanglement or interlacing over a substantial portion of their length. Alternatively, core polymer filaments 4d may be woven, braided, or entangled. Core polymer filaments 4d may be spun inside the sheath polymer 6d. In some embodiments of the invention, as few as about three and as many as about fifty, or more, core polymer filaments 4d may be utilized to form a single fiber 2d.

The sheath polymer may be applied to the core polymer of the bi-component fiber including, for example, by extrusion, co-extrusion, pultrusion, gel spinning with one of the aforementioned processes, melt coating, spray coating, ultrasonic spray coating, electrostatic coating; powder coating, immersion coating by dipping, spraying, solvent evaporation, sheath heat crimping, chemical surface modification, and combinations thereof.

At least a portion of the surface of the core polymer may include some degree of porosity to help anchor or impregnate at least a portion of the sheath polymer into the core. The porosity can be achieved by roughening the at least a portion of the surface of the core polymer. Alternatively, the core polymer may maintain a smooth, non-porous surface such that the core and sheath polymers may show little or no adhesion to each other.

In some embodiments of the invention, the core of any bi-component fiber can provide one or more desirable mechanical properties, e.g., tensile strength, to maintain the integrity of the entire bi-component fiber during use, such as as a wound dressing, while the sheath provides a smooth, non-adherent outer surface for placement against tissue. For example, the core can comprise a material with a greater tensile strength or a greater modulus than the material of the sheath.

In embodiments utilizing multifilament yarns, at least two filaments can be arranged with openings therebetween. Wound dressings comprising multifilament yarns can be arranged relatively in configurations having openings therein. The spacing between the yarns may vary and be predetermined, depending on the medical or surgical application and, in some cases, any desired wound dressing properties.

Multifilament yarns may be heterogeneous or homogeneous yarns. As illustrated in FIG. 6, heterogeneous yarns 10 can include at least two different filaments 12a and 12b, optionally with openings 14 formed between filaments. Homogeneous yarns 10a, as shown in FIG. 7, can include different filaments, or can include similar filaments 12a. Openings 14a can be present between filaments. In embodiments in which at least two filaments form a yarn, the filaments may be drawn (FIG. 6), braided (FIG. 7), or otherwise oriented, crinkled, twisted, commingled or air entangled to form the yarn. The degree or density of openings 14a can be uniform, or can vary along a along a length of the yarn. In other cases, the density of openings can vary as a gradient, e.g., increasing linearly or non-linearly, from one edge or side to another, opposite edge or side. In other cases, the density of openings can vary as a gradient, e.g., increasing or decreasing linearly or non-linearly, from an outer edge or side to an inner or central region. The difference in densities therebetween can be in a range of from about 5% to about 80%, as determined by the number of openings per unit area.

Yarns may include any number of fibers and may be dimensioned in a variety of sizes and shapes. In some particular embodiments of the invention, yarns may have a size in a range of from about 160 dtex to 197 dtex (30 Ne count to about 37 Ne count). (The count of a cotton yarn is the number of hanks, each of 770m (840 yards) in 0.45kg (1 lb).) The yarns in any of the various embodiments of the invention can have a break factor of at least about 1,900 lbNe, at least about 2,000 lb·Ne, and typically in a range of from about 2,000 lb·Ne to about 2,500 lb·Ne (861kg·770m/0.45kg, at least about 907kg·770m/0.45kg, and typically in a range of from about 907kg·770m/0.45kg to about 1134Kg·770m/0.45kg. (1,900lb·Ne, at least about 2,000lb·Ne, and typically in a range of from about 2,000lb·Ne to about 2,500lb·Ne)

The yarns may be braided, twisted, aligned, fused, or otherwise joined to form a variety of different wound dressing configurations. The yarns may be woven, knitted, interlaced, braided, hydroentangled, or combinations thereof, to be formed into a substrate, such as a fabric, for a wound dressing or by other non-weaving techniques. The structure thereof will vary depending upon the assembling technique utilized to form the fabric, as well as other factors such as the type of fibers used, the tension at which the yarns are held, and the mechanical properties required of the wound dressing.

In some embodiments in accordance with some aspects of the invention, knitting may be utilized to form any of the various wound dressings. Knitting typically involves the intermeshing of yarns to form loops, or inter-looping of the yarns. In some embodiments of the invention, any of the various herein disclosed yarns may be warp-knitted thereby creating vertical interlocking loop chains and/or may be weft-knitted thereby creating rows of interlocking loop stitches across the wound dressing.

Weaving may include the intersection of two sets of straight yarns, warp and weft, which cross and interweave at right angles to each other, or the interlacing of two yarns at right angles to each other. In some embodiments, the yarns may be arranged to form a net wound dressing which has isotropic or near isotropic tensile strength and elasticity.

Any of the substrates of the present invention may be formed into a nonwoven substrate by any technique including any of mechanically, chemically, any thermally bonding the yarns into a sheet or web in a random or systematic arrangement. For example, one or more yarns of the present invention may be mechanically bound by entangling the yarns to form the wound dressing by means other than knitting or weaving, such as matting, pressing, stitch-bonding, needlepunching, or otherwise interlocking the yarns to form a binderless network. Any of the yarns may be chemically bound by an adhesive, such as a hot melt adhesive, or be thermally bound by a binder such as a powder, paste, or melt, and melting the binder on the sheet or web of yarns.

Yarns in staple form may be spun using standard spinning methodologies, such as open end spinning, ring spinning, air jet spinning, and other techniques to form any of the substrates.

Any of the wound dressings in accordance with the present invention can include a first set of yarns fabricated from natural and/or man-made cellulosic fibers and a second, or supplementary set of yarns fabricated from alternative polymeric fibers. Alternatively, wound dressings may solely include yarns fabricated from alternative polymeric fibers.

Natural cellulosic materials that may be utilized in any one or more configurations of the invention include, for example, cotton, linen, combinations, and derivatives thereof. Other materials that may be utilized in any one or more configurations of the invention include, for example, bast fibers or other fibers derived from plant stems or barks such as, for example, flax, hemp, jute, ramie, and derivatives thereof. Other materials can include man-made cellulosic materials such as, for example, rayon, rayon acetate, viscose rayon, lyocell, and combinations thereof. Synthetically modified natural polymers of cellulose derivatives may be utilized in any one or more configurations of the invention include, for example, alkyl celluloses, hydroxyalkyl celluloses, cellulose ethers, cellulose esters, nitrocelluloses, and chitosan. Non-limiting examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt. Any of the substrates can incorporate one or more types of fibers. For example, one or more embodiments of the invention can comprise any of cotton, linen, with any one or more bast fibers such as any of flax, hemp, jute, and ramie. Commercially available bast fibers that can be utilized in the various embodiments of the invention include those available as, for example, CRAILAR fibers from Naturally Advanced Technologies Inc., Victoria, British Columbia, Canada.

Alternative polymeric materials that may be utilized in any one or more configurations of the invention include, for example, polyolefins such as polyethylene, including ultra high molecular weight polyethylene, and polypropylene, including atactic, isotactic, syndiotactic, and blends thereof; polyethylene glycols; polyethylene oxides; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; etheylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; spandex; silicones; and copolymers and combinations thereof.

Alternative polymeric fibers that may be utilized in any one or more configurations of the invention may also include blended fibers of alternative polymeric materials, such as any of those described herein. Fibers that may be utilized any one or more aspects of the invention may be a blend of viscose rayon and polyethylene. The fibers may be a blend of lyocell and polyester. The amount of alternative polymeric material in the blended fibers may vary from about 5% to about 65% by weight, in embodiments, from about 10% to about 60% by weight, and in some embodiments, from about 20% to about 50% by weight of the wound dressing. In some cases, the alternative polymeric fibers involve blends with bast fibers or cellulosic fibers.

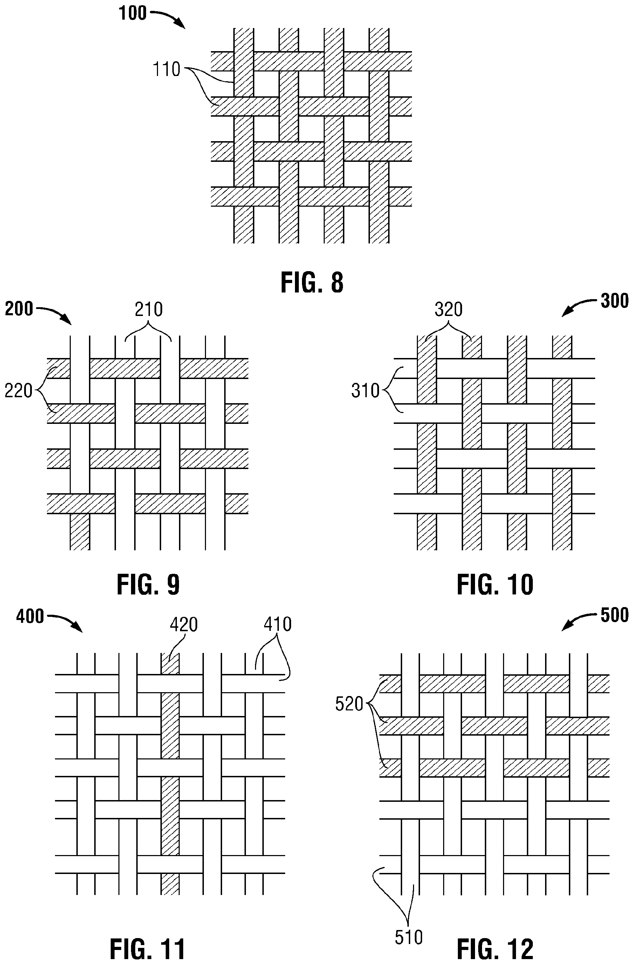

For example, as exemplarily illustrated in FIG. 8, wound dressing 100 can includes yarns 110 including blended fibers of cellulosic and alternative polymeric materials. The yarns 110 may be monofilament or multifilament, homogeneous or heterogeneous, yarns. The yarns 110 may be interconnected in any manner as described herein, such as woven as exemplarily illustrated. The yarns 110 may all be comprised of the same or different blended fibers of bast fibers, cellulosic fibers, or combinations thereof and alternative polymeric materials, in the same or varying mix ratio of bast fibers or cellulosic fibers or combinations thereof to alternative polymeric material. Yarns including bast fibers, cellulosic fibers and alternative polymeric fibers may be used in a variety of configurations to achieve the overall amount of alternative polymeric fibers in a wound dressing in a range of about 5% to about 50% by weight of the wound dressing.

For example, as exemplarily shown in FIG. 9, a wound dressing 200 can include first yarns 210 including bast fibers, cellulosic fibers, or combinations thereof arranged in a warp direction and second yarns 220 including alternative polymeric fibers interlaced between the first yarns 210 in a weft direction to form a weaved pattern. Alternatively, as shown in FIG. 10, a wound dressing 300 may include first yarns 310 including bast fibers, cellulosic fibers or combinations thereof, arranged in a weft direction and second yarns 220 including alternative polymeric fibers in a warp direction. In any of such embodiments, the overall amount of alternative polymeric fibers may be about 50% by weight of the wound dressings.

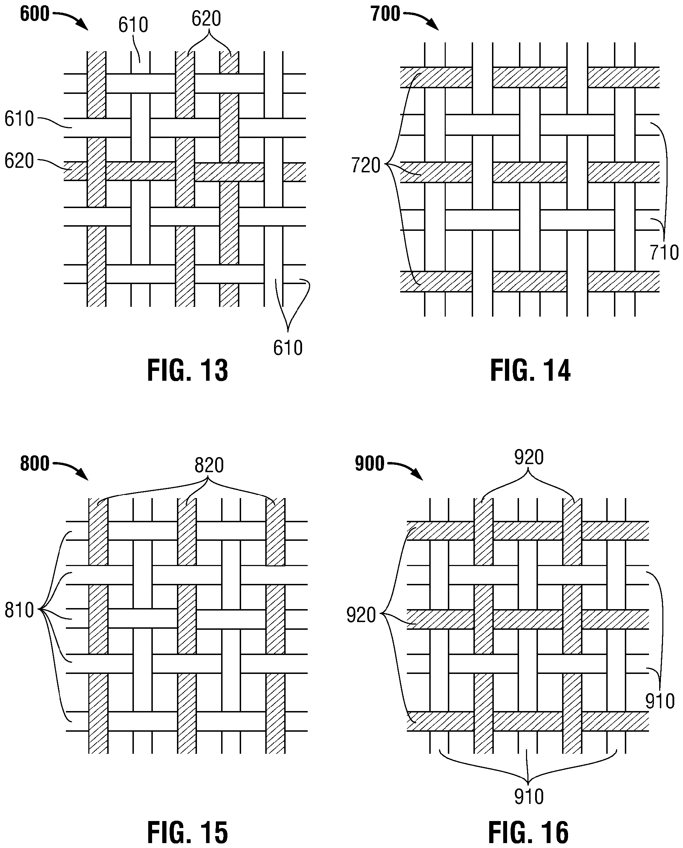

As exemplarily illustrated in FIGS. 11-13, wound dressings 400, 500, and 600, respectively, may include at least one yarn 420 including alternative polymeric fibers in a warp direction (FIG. 11), at least one yarn 520 including alternative polymeric fibers in a weft direction (FIG. 12), or at least one yarn 620 including alternative polymeric fibers in both warp and weft directions (FIG. 13). The remainder of the yarns 410, 510, and 610 can include bast fibers, cellulosic fibers, or combinations thereof. Yarns 410, 510, and 610 can include bast fibers, cellulosic fibers and may include cotton, viscose rayon, and/or blends thereof. The amount of alternative polymer fibers in such embodiments may vary from about 5% to about 50% by weight of the wound dressing.

FIG. 14-16 exemplarily illustrate wound dressings 700, 800, and 900, respectively, including alternating yarns of bast fibers, cellulosic fibers, or combinations thereof and alternate polymeric fibers. As exemplarily shown in FIG. 14, yarns 710 can include bast fibers, cellulosic fibers, or combinations thereof and yarns 720 can include alternative polymeric fibers alternate in a weft direction, with fibers 710 making up the yarns in the warp direction. As shown in FIG. 15, yarns 810 can include bast fibers, cellulosic fibers, or combinations thereof and yarns 820 can include alternative polymeric fibers alternating in a warp direction, with yarns 810 making up the yarns in the weft direction. As shown in FIG. 16, yarns 910 can include bast fibers, cellulosic fibers, or combinations thereof and yarns 920 can include alternative polymer fibers alternating in both warp and weft directions. In any of such variants, the amount of alternative polymeric fibers may be about 25% to about 50% by weight of the wound dressing.

Particular embodiments of the invention can involve embodiments comprising an absorbent gauze comprising cotton, with not more than about 55% by weight of rayon, as a plain woven cloth. Preferably, the absorbent gauze is sterile. Any of the various embodiments of the invention can involve substrates, such as an absorbent gauze, comprising warp threads in a range of from about 41 to about 47 per centimeter, filling threads in a range of 33 to 39 per centimeter. Any of the various embodiments of the invention can involve substrates, such as an absorbent gauze, with an average thread count in a range of about 76 to about 84 threads per 6.45 cm², and basis weight in a range of from about 43.8 to about 55.8 grams per square meter. Any of the various embodiments of the invention can involve substrates, such as an absorbent gauze, comprising warp threads in a range of from about 18 to about 22 per centimeter, filling threads in a range of 8 to 14 per centimeter. Any of the various embodiments of the invention can involve substrates, such as an absorbent gauze, with an average thread count in a range of about 27 to about 35 threads per 6.45 cm², and basis weight in a range of from about 18.1 to about 23.1 grams per square meter. Any of the various embodiments of the invention can involve substrates, such as an absorbent gauze, comprising warp threads in a range of from about 12 to about 16 per centimeter, filling threads in a range of 8 to 12 per centimeter. Any of the various embodiments of the invention can involve substrates, such as an absorbent gauze, with an average thread count in a range of about 21 to about 27 threads per 6.45 cm², and basis weight in a range of from about 12.1 to about 15.5 grams per square meter.

It is envisioned that any number of yarns, in various arrangements and patterns, may be used to form the substrates and wound dressings of the present invention. The yarns, fabrics, or substrates may be scoured and bleached to meet desirable, suggested, and/or mandated standards, such as USP gauze fabric standards. The yarns, fabrics, substrates or wound dressings may be sterilized using standard sterility protocols to conform to suggested or mandated sterility standards. For example, the various embodiments or components thereof of the invention can be sterilized to conform with sterilization standards of medical devices as set forth by the International Organization for Standardization including, for example, any of ISO 11135 for ethylene oxide sterilization for medical devices, ISO 11137 for gamma and e-beam sterilization for medical devices, and ISO 17665 for steam sterilization for medical devices. Sterilizing any of the yarns, substrates, gauze and wound dressings of the invention can involve any suitable technique that provides a desired level of sterility, such as a desired sterility assurance level, including, for example, any one or more of physical processes such as steaming, autoclaving, heating, chemical processes such as exposure to agents such as hydrogen peroxide, ethylene oxide, ozone, silver ions, or other oxidizing compounds such as sodium hypochlorite, irradiation processes such as exposure to gamma rays, electron beams, ultraviolet light and x-ray energy, and combinations thereof.

Bioactive agents, such as medicaments, may be added to a wound dressing of the present disclosure. A "bioactive agent" as used herein, is used in its broadest sense and includes any substance or mixture of substances that have clinical use. A bioactive agent, or combinations thereof, may be utilized to reduce bioburden in a wound, promote healing, and reduce pain associated with changes or removal of the wound dressing. Moreover, any agent which may enhance tissue repair, limit the risk of sepsis, and desirably modulate the mechanical properties of the wound dressing, e.g., the swelling rate and tensile strength, may be added during the preparation of the wound dressing or may be bonded or coated on or into a fiber or yarn; one or more sides of a fiber or yarn; or openings of the wound dressing. The bioactive agent may be applied to the wound dressing utilizing any suitable method known to those skilled in the art. Some examples include, but are not limited to, spraying, dipping, layering, and calendering.

Bioactive agents such as polyhexamethylene biguanide, or any other medicaments, antimicrobials, wound healing agents, and/or wound debriding agents, may be used to decrease the incidence of infection or otherwise promote healing of a wound. Other agents include those used in slow release treatments wherein the agent is released from a fiber or yarn into the wound over a period of time. Other bioactive agents that may utilized in any one or more variant embodiments of the invention can include, for example, a therapeutic agent, an organoleptic agent and a pharmaceutical agent including, for example, an antimicrobial agent, in growth factor, an analgesic, a tissue scaffolding agent, a wound debriding agent, a hemostatic agent, an anti-thrombogenic agent, an anesthetic, an anti-inflammatory agent, an anticancer agent, a vasodilation substance, a wound healing agent, an angiogenic agent, an angiostatic agent, an immune boosting agent, a skin sealing agent, combinations thereof. Any of the one or more bioactive agents may be disposed into any the fibers, yarns, substrates, and wound dressings of the invention by immersion thereof in a solution including the one or more agents, and, optionally, drying the solvents from the immersed, coated, infused fiber, yarn, substrate or wound dressing to any desired bioactive agent concentration, for example, to a concentration that at least partially inhibits any microbial activity therein. Introduction of the one or more bioactive agents can be performed during or after any one or more yarns fabrication, substrates fabrication, or wound dressing fabrication, or, in some cases, after any one of bleaching, and a sterilizing.

Further aspects of the invention can be directed to methods or techniques utilizing the substrates and gauze as wound dressings as disclosed herein to absorb wound exudates, to protect wounds, to cushion wound sites. Such methods and techniques can involve securing any of the wound dressings on the wound or wound site, replacing the wound dressing, and/or reapplying the wound dressing comprising, consisting essentially of, or consisting of the first and second fibers.

It should be understood that the wound dressings of the present disclosure are not limited to those illustrated and described herein and alternate wound dressings and components thereof may be utilized. Moreover, wound dressings of the present invention may be formed by layering one or more of the same or different wound dressings together to form a three-dimensional structure with any one or more desired dressing properties.

## Claims

1. A wound dressing (100, 200, 300, 400, 500, 600, 700, 800, 900) comprising a substrate including first yarns (10, 110, 210, 310, 410, 510, 610, 710, 810, 910) and second yarns (10, 120, 220, 320, 420, 520, 620, 720, 820, 920),
the first yarns including at least one fiber (12a, 12b) comprising at least one of a cellulosic material or a bast fiber, and
the second yarns including at least one fiber (2a, 2b, 2c, 2d, 12a, 12b) comprising an alternative polymeric material,
wherein at least one of the first and second yarns includes at least one monofilament bi-component fiber (2, 2a, 2b, 2c, 2d) fabricated by co-extruding two distinct polymers.

2. The wound dressing of claim 1, wherein at least a portion of the first yarns are heterogeneous yarns including at least one fiber of a first cellulosic material and at least one fiber of a second cellulosic material.

3. The wound dressing of claim 1, wherein at least a portion of the first yarns includes at least one fiber comprising a fiber material selected from the group consisting of cotton, rayon, flax, lyocell, and combinations thereof.

4. The wound dressing of claim 1, wherein at least a portion of the second yarns are heterogeneous yarns including at least one fiber of a first alternative polymeric material and at least one fiber of a second alternative polymeric material.

5. The wound dressing of claim 1, wherein at least a portion of the second yarns includes at least one fiber comprising an alternative polymeric material selected from the group consisting of polyolefins, polyamides, polyesters, and combinations thereof.

6. The wound dressing of claim 1, wherein at least a portion of the second yarns includes at least one blended fiber comprising a cellulosic material and an alternative polymeric material.

7. The wound dressing of claim 6, wherein an amount of the alternative polymeric material is in a range of from 5% to 65% by weight of the substrate.

8. The wound dressing of claim 1, wherein the substrate comprises a woven fabric with at least a portion of the first yarns woven in a warp direction and at least a portion of the second yarns woven a weft direction.

9. The wound dressing of claim 1, wherein the substrate comprises a woven fabric with at least a portion of the first yarns woven in a weft direction and at least a portion of the second yarns woven a warp direction.

10. A method of providing a wound dressing (100, 200, 300, 400, 500, 600, 700, 800, 900) comprising:
providing a wound dressing including first yarns (10, 110, 210, 310, 410, 510, 610, 710, 810, 910) and second yarns (10, 120, 220, 320, 420, 520, 620, 720, 820, 920),
the first yarns including at least one fiber (12a, 12b) comprising one or more of a material selected from the group consisting of cotton, rayon, flax, lyocell, and combinations thereof, and
the second yarns including at least one fiber (2a, 2b, 2c, 2d, 12a, 12b) comprising an alternative polymeric material selected from the group consisting of polyolefins, polyamides, polyesters, and combinations thereof,
wherein at least one of the first and second yarns includes at least one monofilament bi-component fiber (2, 2a, 2b, 2c, 2d) fabricated by co-extruding two distinct polymers; and
sterilizing the wound dressing.

11. The method of claim 10, wherein providing the wound dressing comprises weaving at least a portion of the first yarns in a weft direction and at least a portion of the second yarns in a warp direction to produce a fabric with an amount of the alternative polymeric material in a range of from 5% to 65% by weight thereof.

12. The method of claim 10, wherein providing the wound dressing comprises weaving at least a portion of the first yarns in a warp direction and at least a portion of the second yarns in a weft direction to produce a fabric with an amount of the alternative polymeric material in a range of from 5% to 65% by weight thereof.

13. The method of claim 10, wherein providing the wound dressing comprises weaving at least a portion of the first yarns consisting essentially of heterogeneous yarns (10, 110, 210, 310, 410, 510, 610, 710, 810, 910) with at least one fiber of a first cellulosic material and at least one fiber of a second cellulosic material with at least a portion of the second yarns consisting essentially of heterogeneous yarns with at least one fiber of a first alternative polymeric material and at least one fiber of a second alternative polymeric material to produce a fabric with an amount of the alternative polymeric material in a range of from 5% to 65% by weight thereof.

14. The method of claim 13 wherein providing the wound dressing comprises:
weaving at least a portion of the first yarns with the second yarns to produce a woven article;
bleaching the woven article;
tentering the bleached, woven article;
cutting the bleached, woven article to produce a woven fabric; and
folding the woven fabric to produce a gauze.

15. The method of claim 14 wherein folding the woven fabric comprises folding the woven fabric to produce a gauze having any of three plies, four plies, five plies, six plies, eight plies, ten plies, twelve plies, sixteen plies, 24 plies, 32 plies, 48 plies, 50 plies, 144 plies, and 216 plies.

16. The method of claim 10, wherein providing the wound dressing comprises weaving first yarns consisting essentially of cotton with second yarns consisting essentially of polyester to produce a fabric with an amount of the polyester in a range of from 5% to 65% by weight of the fabric.

17. The method of claim 15, wherein providing the wound dressing comprises weaving the first yarns in a warp direction of the fabric.

18. The method of claim 15, wherein providing the wound dressing comprises weaving the first yarns in a weft direction of the fabric.

## Patentansprüche

1. Wundverband (100, 200, 300, 400, 500, 600, 700, 800, 900) umfassend ein Substrat, das erste Garne (10, 110, 210, 310, 410, 510, 610, 710, 810, 910) und zweite Garne (10, 120, 220, 320, 420, 520, 620, 720, 820, 920) umfasst,
wobei die ersten Garne mindestens eine Faser (12a, 12b) umfassen, die mindestens eines von einem cellulosischen Material oder einer Bastfaser umfasst und
die zweiten Garne mindestens eine Faser (2a, 2b, 2c, 2d, 12a, 12b) umfassen, die ein alternatives polymeres Material umfasst,
wobei mindestens eines der ersten und zweiten Garne mindestens eine Monofilament-Bi-Komponentenfaser (2, 2a, 2b, 2c, 2d) umfasst, die durch Coextrudieren von zwei verschiedenen Polymeren hergestellt wird.

2. Wundverband nach Anspruch 1, wobei mindestens ein Teil der ersten Garne heterogene Garne sind, die mindestens eine Faser aus einem ersten cellulosischen Material und mindestens eine Faser aus einem zweiten Cellulosematerial umfassen.

3. Wundverband nach Anspruch 1, wobei mindestens ein Teil der ersten Garne mindestens eine Faser umfasst, die ein Fasermaterial umfasst ausgewählt aus der Gruppe bestehend aus Baumwolle, Rayon, Flachs, Lycell und Kombinationen davon.

4. Wundverband nach Anspruch 1, wobei mindestens ein Teil der zweiten Garne heterogene Garne sind, die mindestens eine Faser aus einem ersten alternativen polymeren Material und mindestens eine Faser aus einem zweiten alternativen polymeren Material umfassen.

5. Wundverband nach Anspruch 1, wobei mindestens ein Teil der zweiten Garne mindestens eine Faser umfasst, die ein alternatives polymeres Material umfasst ausgewählt aus der Gruppe bestehend aus Polyolefinen, Polyamiden, Polyestern und Kombinationen davon.

6. Wundverband nach Anspruch 1, wobei mindestens ein Teil der zweiten Garne mindestens eine Mischfaser umfasst, die ein cellulosisches Material und ein alternatives polymeres Material umfasst.

7. Wundverband nach Anspruch 6, wobei die Menge des alternativen polymeren Materials in einem Bereich von 5 bis 65 Gew.-% des Substrats liegt.

8. Wundverband nach Anspruch 1, wobei das Substrat einen gewobenen Textilstoff umfasst, wobei mindestens ein Teil der ersten Garne in einer Kettenrichtung und mindestens ein Teil der zweiten Garne in einer Schussrichtung gewoben sind.

9. Wundverband nach Anspruch 1, wobei das Substrat einen gewobenen Textilstoff umfasst, wobei mindestens ein Teil der ersten Garne in einer Schussrichtung und mindestens ein Teil der zweiten Garne in einer Kettenrichtung gewoben sind.

10. Verfahren zum Bereitstellen eines Wundverbands (100, 200, 300, 400, 500, 600, 700, 800, 900) umfassend:
Bereitstellen eines Wundverbands, der erste Garne (10, 110, 210, 310, 410, 510, 610, 710, 810, 910) und zweite Garne (10, 120, 220, 320, 420, 520, 620, 720, 820, 920) umfasst,
wobei die ersten Garne mindestens eine Faser (12a, 12b) umfassen, die eines oder mehrere von einem Material umfassen ausgewählt aus der Gruppe bestehend aus Baumwolle, Rayon, Flachs, Lycell und Kombinationen davon und
wobei die zweiten Garne mindestens eine Faser (2a, 2b, 2c, 2d, 12a, 12b) umfassen, die ein alternatives polymeres Material umfasst, ausgewählt aus der Gruppe bestehend aus Polyolefinen, Polyamiden, Polyestern, und Kombinationen davon.
wobei mindestens eines der ersten und zweiten Garne mindestens eine Monofilament-Bi-Komponentenfaser (2, 2a, 2b, 2c, 2d) umfasst, die durch Coextrudieren von zwei verschiedenen Polymeren hergestellt wird; und
Sterilisieren des Wundverbands.

11. Verfahren nach Anspruch 10, wobei das Bereitstellen des Wundverbands das Weben mindestens eines Teils der ersten Garne in einer Schussrichtung und mindestens eines Teil der zweiten Garne in einer Kettenrichtung umfasst, um einen Textilstoff mit einer Menge an alternativem polymerem Material in einem Bereich von 5 bis 65 Gew.-% davon herzustellen.

12. Verfahren nach Anspruch 10, wobei das Bereitstellen des Wundverbands das Weben mindestens eines Teils der ersten Garne in einer Kettenrichtung und mindestens eines Teil der zweiten Garne in einer Schussrichtung umfasst, um einen Textilstoff mit einer Menge an alternativem polymerem Material in einem Bereich von 5 bis 65 Gew.-% davon herzustellen.

13. Verfahren nach Anspruch 10, wobei das Bereitstellen des Wundverbands das Weben mindestens eines Teils der ersten Garne, die im Wesentlichen aus heterogenen Garnen (10, 110, 210, 310, 410, 510, 610, 710, 810, 910) mit mindestens einer Faser aus einem ersten cellulosischen Material und mindestens einer Faser aus einem zweiten cellulosischen Material bestehen, wobei mindestens ein Teil der zweiten Garne im Wesentlichen aus heterogenen Garnen mit mindestens einer Faser aus einem ersten alternativen polymeren Material und mindestens einer Faser aus einem zweiten alternativen polymeren Material besteht, um einen Textilstoff mit einer Menge des alternativen polymeren Materials in einem Bereich von 5 bis 65 Gew.-% davon herzustellen.

14. Verfahren nach Anspruch 13, wobei das Bereitstellen des Wundverbands Folgendes umfasst:
Weben mindestens eines Teils der ersten Garne mit den zweiten Garnen, um einen gewobenen Artikel herzustellen;
Bleichen des gewobenen Artikels;
Aufrahmen des gebleichten, gewobenen Artikels;
Schneiden des gebleichten, gewobenen Artikels, um einen gewobenen Textilstoff herzustellen; und
Falten des gewobenen Textilstoffs, um eine Gaze herzustellen.

15. Verfahren nach Anspruch 14, wobei das Falten des gewobenen Textilstoffs das Falten des gewobenen Textilstoffs zum Herstellen einer Gaze umfasst, die irgendeine von drei Lagen, vier Lagen, fünf Lagen, sechs Lagen, acht Lagen, zehn Lagen, zwölf Lagen, sechzehn Lagen, 24 Lagen, 32 Lagen, 48 Lagen, 50 Lagen, 144 Lagen und 216 lagen aufweist.

16. Verfahren nach Anspruch 10, wobei das Bereitstellen des Wundverbands das Weben erster Garne, die im Wesentlichen aus Baumwolle bestehen, mit zweiten Garnen, die im Wesentlichen aus Polyester bestehen, umfasst, um einen Textilstoff mit einer Menge des Polyesters in einem Bereich von 5 bis 65 Gew.-% des Textilstoffs herzustellen.

17. Verfahren nach Anspruch 15, wobei das Bereitstellen des Wundverbands das Weben der ersten Garne in einer Kettenrichtung des Textilstoffs umfasst.

18. Verfahren nach Anspruch 15, wobei das Bereitstellen des Wundverbands das Weben der ersten Garne in einer Schussrichtung des Textilstoff umfasst.

## Revendications

1. Pansement pour plaie (100, 200, 300, 400, 500, 600, 700, 800, 900) comprenant un substrat comprenant des premiers fils (10, 110, 210, 310, 410, 510, 610, 710, 810, 910) et des seconds fils (10, 120, 220, 320, 420, 520, 620, 720, 820, 920),
les premiers fils comprenant au moins une fibre (12a, 12b) comprenant au moins l'un d'un matériau cellulosique ou d'une fibre de liber, et
les seconds fils comprenant au moins une fibre (2a, 2b, 2c, 2d, 12a, 12b) comprenant un matériau polymère alternatif,
au moins l'un des premiers et des seconds fils comprenant au moins une fibre bi-composée monofilament (2, 2a, 2b, 2c, 2d) fabriquée par co-extrusion de deux polymères distincts.

2. Pansement pour plaie selon la revendication 1, au moins une partie des premiers fils étant des fils hétérogènes comprenant au moins une fibre d'un premier matériau cellulosique et au moins une fibre d'un second matériau cellulosique.

3. Pansement pour plaie selon la revendication 1, au moins une partie des premiers fils comprenant au moins une fibre comprenant un matériau fibreux sélectionné dans le groupe constitué du coton, de la rayonne, du lin, du lyocell, et de leurs combinaisons.

4. Pansement pour plaie selon la revendication 1, au moins une partie des seconds fils étant des fils hétérogènes comprenant au moins une fibre d'un premier matériau polymère alternatif et d'au moins une fibre d'un second matériau polymère alternatif.

5. Pansement pour plaie selon la revendication 1, au moins une partie des seconds fils comprenant au moins une fibre comprenant un matériau polymère alternatif sélectionné dans le groupe constitué des polyoléfines, des polyamides, des polyesters, et de leurs combinaisons.

6. Pansement pour plaie selon la revendication 1, au moins une partie des seconds fils comprenant au moins une fibre mélangée comprenant un matériau cellulosique et un matériau polymère alternatif.

7. Pansement pour plaie selon la revendication 6, une quantité du matériau polymère alternatif se situant dans une plage de 5 % à 65 % en poids du substrat.

8. Pansement pour plaie selon la revendication 1, le substrat comprenant un tissu tissé avec au moins une partie des premiers fils tissée dans un sens de la chaîne et au moins une partie des seconds fils tissée dans un sens de la trame.

9. Pansement pour plaie selon la revendication 1, le substrat comprenant un tissu tissé avec au moins une partie des premiers fils tissée dans un sens de la trame et au moins une partie des seconds fils tissée dans un sens de la chaîne.

10. Procédé de préparation d'un pansement pour plaie (100, 200, 300, 400, 500, 600, 700, 800, 900) comprenant:
la préparation d'un pansement pour plaie comprenant de premiers fils (10, 110, 210, 310, 410, 510, 610, 710, 810, 910) et de seconds fils (10, 120, 220, 320, 420, 520, 620, 720, 820, 920),
les premiers fils comprenant au moins une fibre (12a, 12b) comprenant un ou plusieurs d'un matériau sélectionné dans le groupe constitué du coton, de la rayonne, du lin, du lyocell, et de leurs combinaisons, et
les seconds fils comprenant au moins une fibre (2a, 2b, 2c, 2d, 12a, 12b) comprenant un matériau polymère alternatif sélectionné dans le groupe constitué des polyoléfines, des polyamides, des polyesters, et de leurs combinaisons,
au moins l'un des premiers et des seconds fils comprenant au moins une fibre bi-composée monofilament (2, 2a, 2b, 2c, 2d) fabriquée par co-extrusion de deux polymères distincts; et
la stérilisation du pansement pour plaie.

11. Procédé selon la revendication 10, la préparation du pansement pour plaie comprenant le tissage d'au moins une partie des premiers fils dans un sens de la trame et d'au moins une partie des seconds fils dans un sens de la chaîne pour produire un tissu avec une quantité du matériau polymère alternatif située dans une plage de 5 % à 65 % en poids de celui-ci.

12. Procédé selon la revendication 10, la préparation du pansement pour plaie comprenant le tissage d'au moins une partie des premiers fils dans un sens de la chaîne et d'au moins une partie des seconds fils dans un sens de la trame pour produire un tissu avec une quantité du matériau polymère alternatif située dans une plage de 5 % à 65 % en poids de celui-ci.

13. Procédé selon la revendication 10, la préparation du pansement pour plaie comprenant le tissage d'au moins une partie des premiers fils constituée essentiellement de fils hétérogènes (10, 110, 210, 310, 410, 510, 610, 710, 810, 910) avec au moins une fibre d'un premier matériau cellulosique et d'au moins une fibre d'un second matériau cellulosique avec au moins une partie des seconds fils constituée essentiellement de fils hétérogènes avec au moins une fibre d'un premier matériau polymère alternatif et au moins une fibre d'un second matériau polymère alternatif pour produire un tissu avec une quantité du matériau polymère alternatif située dans une plage de 5 % à 65 % en poids de celui-ci.

14. Procédé selon la revendication 13, la préparation du pansement pour plaie comprenant:
le tissage d'au moins une partie des premiers fils avec les seconds fils pour produire un article tissé;
la décoloration de l'article tissé;
l'étirage de l'article tissé, décoloré;
la découpe de l'article tissé, décoloré pour produire un tissu tissé; et
le pliage du tissu tissé pour produire une gaze.

15. Procédé selon la revendication 14, le pliage du tissu tissé comprenant le pliage du tissu tissé pour produire une gaze ayant n'importe lesquels de trois plis, quatre plis, cinq plis, six plis, huit plis, dix plis, douze plis, seize plis, 24 plis, 32 plis, 48 plis, 50 plis, 144 plis, et 216 plis.

16. Procédé selon la revendication 10, la préparation du pansement pour plaie comprenant le tissage de premiers fils constitués essentiellement de coton avec de seconds fils constitués essentiellement de polyester pour produire un tissu ayant une quantité du polyester située dans une plage de 5 % à 65 % en poids du tissu.

17. Procédé selon la revendication 15, la préparation du pansement pour plaie comprenant le tissage des premiers fils dans un sens de la chaîne du tissu.

18. Procédé selon la revendication 15, la préparation du pansement pour plaie comprenant le tissage des premiers fils dans un sens de la trame du tissu.
